# EUROPEAN PATENT SPECIFICATION

(11) **EP 2 675 309 B1**
(45) Date of publication and mention of the grant of the patent: **16.04.2014**
(21) Application number: 11714853.6
(22) Date of filing: 14.02.2011
(51) Int. Cl.: A42B 3/28

(54) **SURGICAL HELMET**
CHIRURGISCHER HELM
CASQUE CHIRURGICAL

(43) Date of publication of application: 25.12.2013
(73) Proprietor: T.H.I. Total Healthcare Innovation GmbH, 9181 Feistritz im Rosental (AT)
(72) Inventor: ROSATI, Giorgio, I-00030 San Cesareo (RM) (IT); VAGLIVIELLO, Marco, 9020 Klagenfurt am Wörthersee (AT); KOGLER, Franz, 9020 Klagenfurt am Wörthersee (AT); TRIPOLT, Stefan, 9020 Klagenfurt am Wörthersee (AT)
(74) Representative: Papa, Elisabetta
(86) International application number: PCT/IT2011/000036
(87) International publication number: WO 2012/111030

(56) References cited:
- US-A- 4 901 716
- US-A- 5 009 225
- US-A- 5 046 492
- US-A- 5 592 936
- US-A- 5 887 281

## Description

### Field of the invention

The present invention refers to a protection device apt to be worn on by a health operator, typically a surgeon, during surgery, and in particular to a device substantially in the form of a helmet.

### Background of the invention

Over the last two decades, protection systems for protecting a health operator's head and face based on a helmet to be worn on during surgery have become of widespread use. Traditionally, such systems are mainly formed just by a helmet, a cap or a protection gown covering the helmet, by a lens - i.e., a transparent splash guard visor - constrained to the cap or gown, by a drive unit and a related power-supplying battery. As mentioned, the system is worn on by the health operator during surgery.

These systems are especially used in orthopedic surgery, with specific reference to surgery for implanting knee and hip prostheses, in which drills provided with reamers and saws are used. In that sense, helmets prove superior to masks and common face protection shields, as the former cover the entire head of the health operator, creating a sterile barrier between the health operator and the patient; said barrier protects the operator from the considerable amount of possibly contaminated blood spurts emitted in the course of surgery.

Moreover, the helmet generally comprises a fan for air circulation inside the environment accommodating the surgeon's head. Such air circulation opposes perspiration and contributes to keep refrigerated the air inside the facial chamber, thereby increasing the operator's comfort level.

In addition, air circulation also offers a valid protection against the so-called "aerosol effect" of virus-contaminated particles. Potential infection risks for the surgeon associated to the aerosol effect and benefits from the use of surgical helmets are amply demonstrated in the literature (see, e.g.: Jonathan A. Eandi et al "Use of a surgical helmet system to minimize splash injury during percutaneous renal surgery in high-risk patients" Journal of Endourology, Vol. 22, No. 12, Dec. 2008).

Moreover, the above-mentioned systems offers a valid protection for the patient as well, with respect to contaminations coming from the surgeon and other health operators, as preventing the fall of hair, dandruff and saliva droplets and therefore the possibility of wound infection. Infection rates described in the literature are of between 0.38% and 2% for THA (Total Hip Arthroplasty) and between 0.77% and 4% for TKA (Total Knee Arthroplasty), data increasing in the course of revision surgery.

Therefore, for all purposes the above-mentioned protection system based on a surgical helmet may be deemed to be both a medical device, owing to the protection offered to the patient, and an individual protection device for the health operator.

### Main drawbacks of the known art

The above-described known protection systems suffer from some relevant drawbacks.

First of all, a mere ventilation of the head-accommodating environment is useless to prevent carbon dioxide accumulation inside the same environment and does not effectively oppose the lens fogging phenomenon, related above all to the health operator's breathing. In connection to this latter aspect, the Inventors observed that only in the first stages of the surgery such a fogging is reduced by means of fan-produced air circulation.

However, as time passes - a hip or knee prosthesis surgery can last up to several hours - besides fogging the above-mentioned CO₂ accumulation occurs, which may be a source of queasiness. In fact, the cap or robe associated to the helmet "seal" the environment at the neck level, allowing no adequate CO₂ evacuation below the gown. To this end, it should be noted that for the manufacturing of the robe or cap the evolution of the field leads to the use of repellent materials, in particular polypropylene ones, preventing perspiration.

Moreover, the known systems have remarkable weights and encumbrances even at the level of the sole helmet (which is then to be associated to lens, motor and battery), penalizing the health operator's comfort at the head level and accordingly limiting his/her body motions.

Furthermore, in the known systems the cap-lens unit is kept in position on the helmet by the Velcro® arranged on the lens and on the stationary structure of the helmet. This complicates the undressing modes of the health operator, who should separate the coupled strips by tearing them off, and may be cause of scarce accuracy in the position of the entire protection system, and specifically of the lens in the dressing stage, since the strips may adhere accidentally according to a coupling configuration different from the desired one.

US 5,046,492 discloses a surgical helmet comprising suction means according to the preamble of claim 1.

### Summary of the invention

Therefore, the technical problem set and solved by the present invention is that of providing a protection system wearable by a health operator during surgery allowing to overcome the drawbacks mentioned above with reference to the known art.

Such a problem is solved by a protection device according to claim 1 and by a protection system according to claim 27.

The present invention provides some relevant advantages. The main advantage lies in the fact that the presence of air suction means operating within the environment accommodating the health operator's head allows an evacuation of exhausted air from said environment, preventing CO₂ accumulation therein.

Said suction means also allows a drastic reduction of the lens fogging phenomenon for the entire duration of the surgery.

Preferred features of the present invention are set forth in the dependent claims thereof.

In particular, according to a particularly preferred feature the device of the invention comprises a helmet formed by structural members having a tubular configuration, i.e. an internally hollow profile.

This allows to attain a maximization of the weight/use ratio of the support structure, increasing the surgeon's comfort and therefore the safety of the surgery. Moreover, such structural members are apt to perform a function of guiding or piping the air flow generated by the suction means.

Moreover, according to another particularly preferred feature coupling flanges are provided, obtained on the helmet, to the ends of a direct connection between the latter and the garment (cap, gown, robe, etc.) associated to the vision lens.

Other advantages, features, and the operation steps of the present invention will be made apparent in the following detailed description of some embodiments thereof, given by way of example and not for limitative purposes.

### Brief Description of the Figures

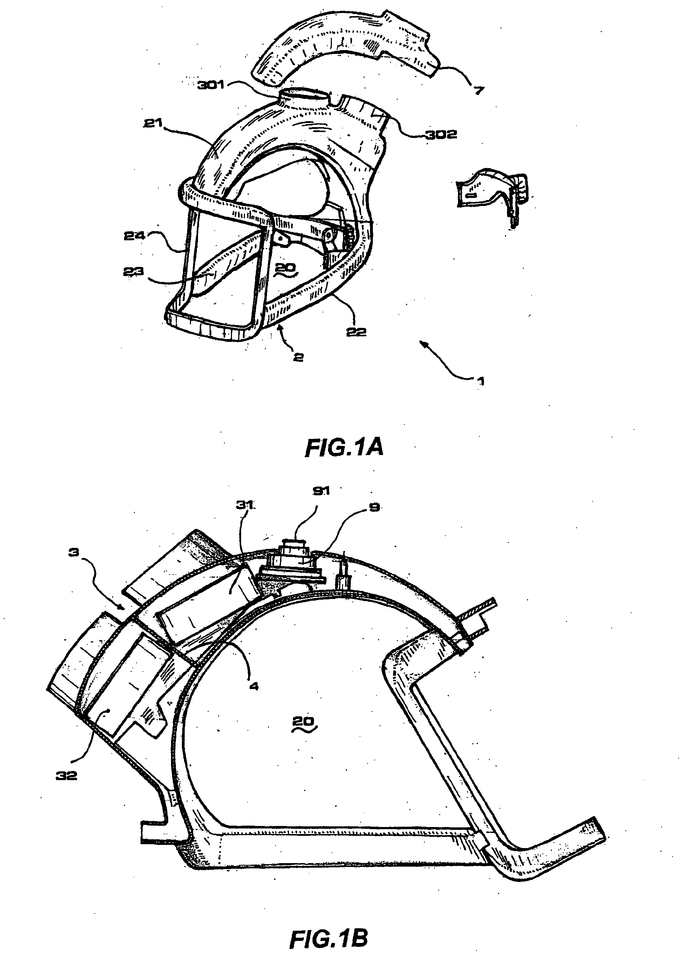

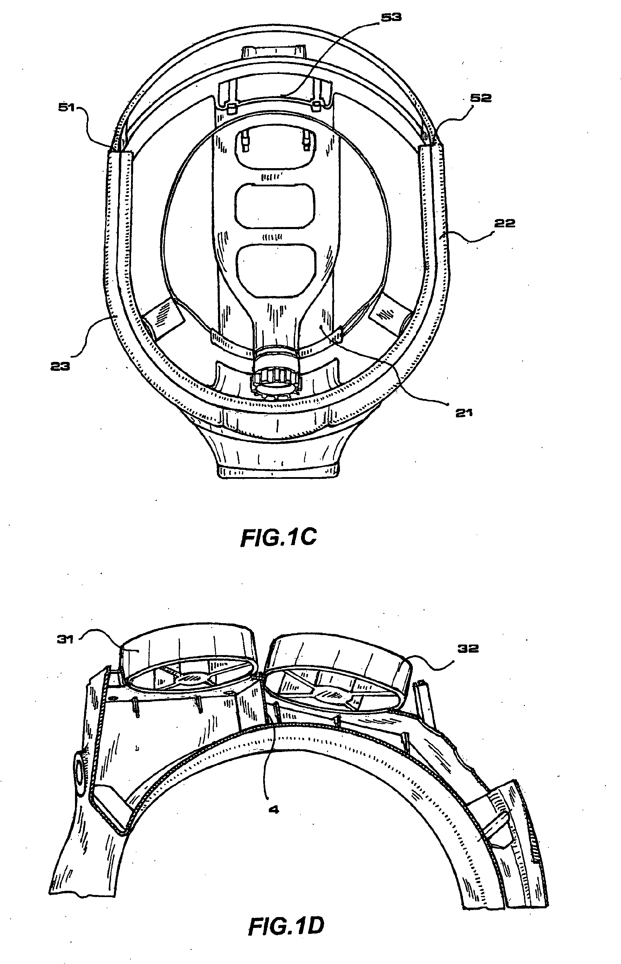

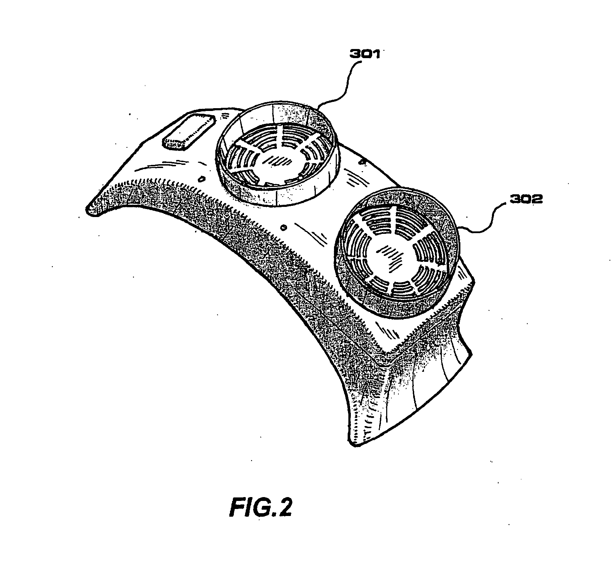

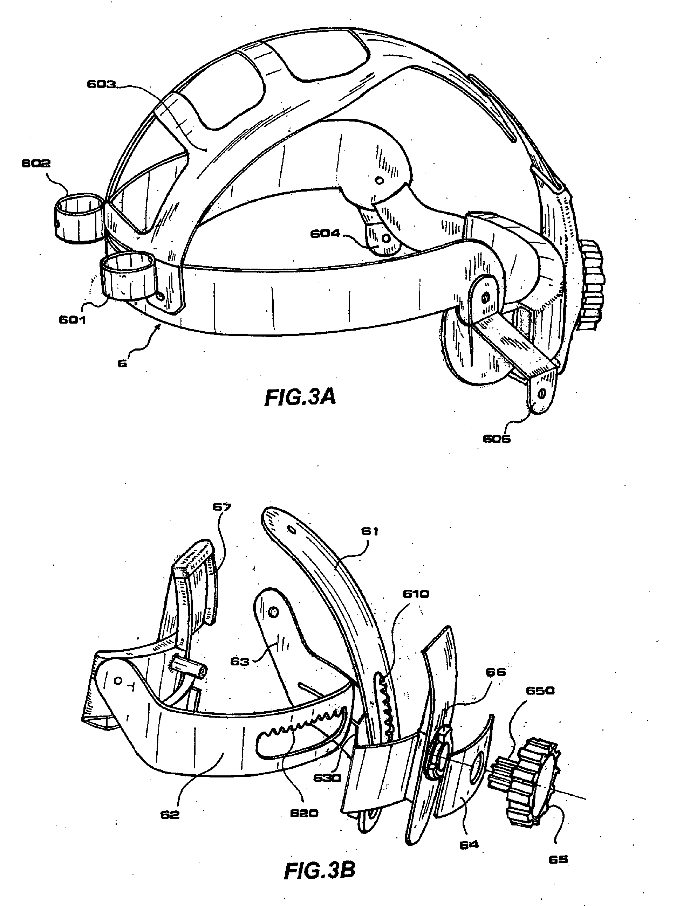

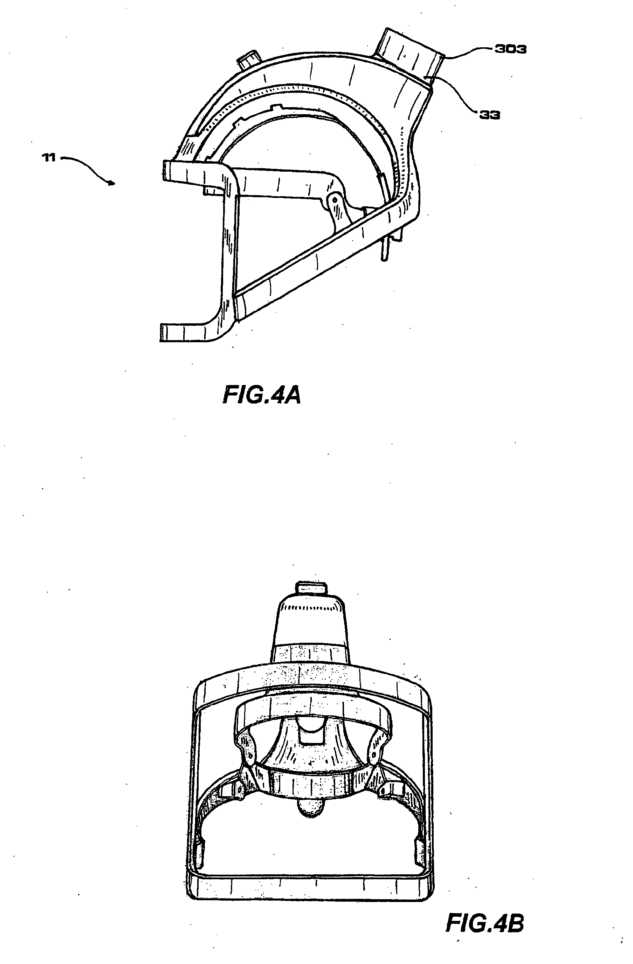

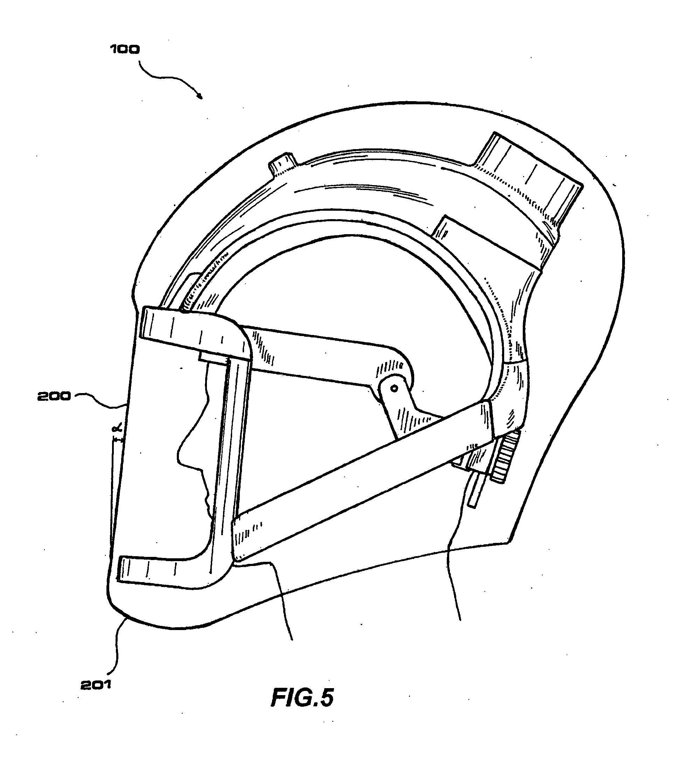

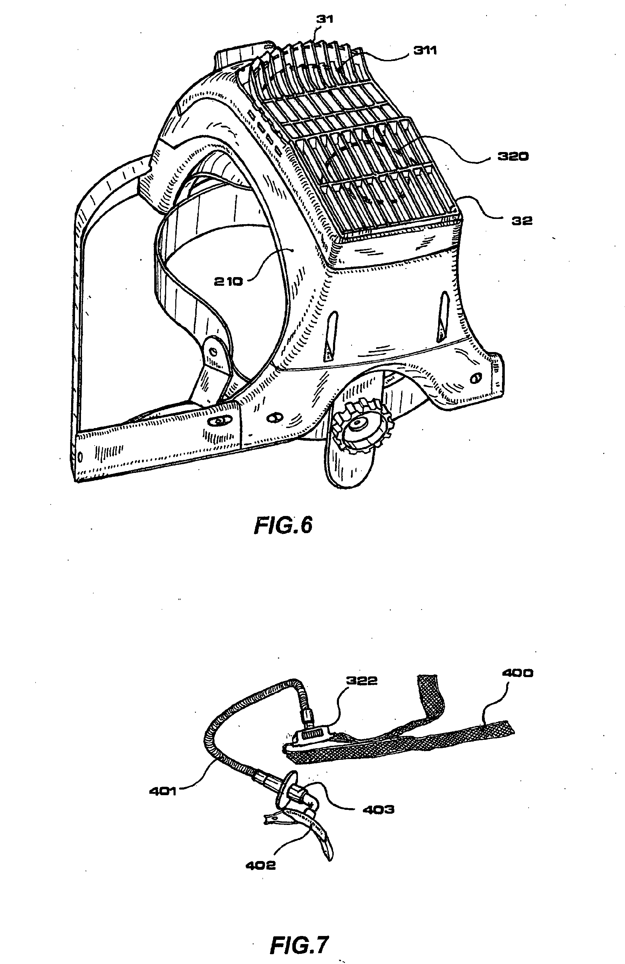

Reference will be made to the figures of the annexed drawings, wherein:
■ Figures 1A, 1B and 1C refer to a first preferred embodiment of the protection device according to the present invention, respectively showing a front perspective view, a longitudinal section view and a bottom plan view thereof;
■ Figure 1D shows a partially sectional side perspective view of a top portion of the device of Figure 1A;
■ Figure 2 shows a top perspective view of a component of the device of Figure 1A;
■ Figures 3A and 3B refer to further internal components of the device of Figure 1A, apt to allow an adjustment of the position of the device itself on the operator's head, showing respectively a side perspective view and an exploded view thereof;
■ Figures 4A and 4B refer to a second preferred embodiment of the protection device according to the present invention, showing respectively a side view and a front view thereof;
■ Figure 5 shows a side view of a protection system incorporating the device of Figure 4A worn on by a health operator;
■ Figure 6 refers to a third preferred embodiment of the protection device according to the present invention, showing a rear perspective view thereof; and
■ Figure 7 refers to a fourth preferred embodiment of the protection device according to the present invention, showing a perspective view of some specific components thereof.

### Detailed description of preferred embodiments

Referring initially to Figures 1A, 1B, 1C and 1D, a protection device apt to be worn on by a health operator, typically a surgeon, during surgery according to a first preferred embodiment of the invention is generally denoted by 1.

The device 1 has a main body 2 substantially shaped as a helmet and apt to surround the health operator's head. Therefore, for simplicity's sake hereinafter the device 1 could also be referred to as surgical helmet.

The main body 2 has a load-bearing structure made with longitudinal and transversal members integral to each other and lightening compartments interposed thereamong. In particular, the main body 2 has a longitudinal upright 21 of curved shape which substantially follows the profile of the operator's head along the sagittal plane of the latter. The longitudinal upright 21 is joined, at a bottom end thereof arranged, in use, substantially in the occipital rear region of the skull, with a pair of crosspieces 22 and 23. The crosspieces extend just laterally to the head, following its contour in an anteroposterior direction, therefore them also developing along a curved profile and defining a bottom part of the main body 2.

The crosspieces 22 and 23 are frontally joined to a further front structural member 24 shaped substantially like a polygonal mask, and in particular substantially rectangular. Such structural mask-like member 24 is joined topwise to the other longitudinal (front) end of the upright 21. The front mask 24 defines a central opening apt to be closed by a transparent vision element, or lens, 200, which is worn on jointly to the helmet 1 and will be described hereinafter.

The surgical helmet 1 further comprises means for forced circulation of air, generally denoted by 3, in an environment, denoted by 20, housing the surgeon's head and defined and externally closed by the main body 2, by the above-mentioned lens 200 and a garment like a cap, robe, gown or the like, to which the lens itself is integral and that will it also be described hereinafter.

In the present example, the means 3 for forced circulation of air comprises a first and a second ventilation means, respectively 31 and 32, typically implemented by axial or radial blowers of a type known per se and housed at an internal portion of the longitudinal upright 21. Such first and second ventilation means 31 and 32 are associated to power supply means, e.g. batteries, not shown in the figures and optionally arranged in a remote position with respect to the main body 2.

The first ventilation means 31 is a means for inletting "fresh" air into the environment 20. According to the invention, the second ventilation means 32 is instead a means for the suction of exhausted air from the environment 20, hence allowing an evacuation of exhausted air from said environment and, therefore, a reduction in CO₂ content.

On the external part of the upright 21, the forced circulation means 3 provide a pair of projections in the form of coupling flanges, each located at a respective inletting 31 or suction 32 means and in turn respectively denoted by 301 and 302.

As best seen in Figure 2, the ventilation means 31, 32 and the related coupling flanges 301, 302 are arranged longitudinally side-by-side in pairs on the upright 21.

As will also be illustrated hereinafter, the flanges 301 and 302 are suitable to allow a direct connection between the surgical helmet 1 and the garment (cap, gown, robe, etc.) associated to the vision lens 200.

In the present example, the coupling flanges 301 and 302 are in the form of hollow cylindrical members.

A variant embodiment may envisage one or both of the active ventilation means 31 and 32 to be at least partially placed in a remote position with respect to the main body 2 and in fluid communication with the environment 20 internal to the latter. Such communication may be established at vents or sleeves associated to or obtained on the main body 2 and identifiable also with the same flanges 301 and 302 introduced hereto. Such a fluid communication may be implemented by pipes, connectors or equivalent members known per se and preferably of snap coupling type on the vents themselves.

Moreover, always according to variant embodiments, one or both of the above-mentioned ventilation means 31 e 32 and their components may also be optionally at least partially carried by the health operator, e.g. at his/her waist, as will be illustrated hereinafter in connection to the embodiment shown in Figure 7.

According to another variant embodiment, a single ventilation means may be provided, apt to alternatively act as air inletting means and as air suction means during a same surgery, by periodically inverting its operation modes.

Referring again to the embodiment of Figures 1A- 1D and 2, preferably control means is provided for controlling the flow rate and/or the velocity of air inlet and/or sucked by the ventilation means 31 and 32. In the present example such control means is embedded in a control unit 9, preferably positioned at an apical portion of the upright 21, forward to the ventilation means 31, 32. Preferably, the control unit 9 provides a sequential logic of operation.

Advantageously, and as best seen in Figure 1B, the control unit 9 comprises a top actuation element 91, e.g. a pushbutton, or a slidable or rotatably slider. The arrangement described is such that said actuation element 91 is in an ergonomic position easily accessible by the operator or his/her collaborators, even at the dressing stage.

As best seen in Figure 1C, to foster forced air circulation inside the environment 20 the load-bearing structural members 21, 22 and 23 defining the main body 2 preferably have a tubular configuration, in the sense of being internally hollow, and are in communication with each other. Thus, they are apt to guide the air flow generated by the forced circulation means 3. The side structural members 22 and 23 are in communication with the environment 20, each by a respective front opening or slit 51 and 52, whereas the top structural member is in communication with said environment 20 at its own front opening or slit 53. Moreover, the side members 22 and 23 are in communication therebetween and with the longitudinal upright 21 at a rear base of the latter. Therefore, such members 21, 22 and 23 define one or more chambers, internal to the main body 2 and apt to allow air circulation therein and in the environment 20 as will be detailed hereinafter.

As already highlighted, the above-mentioned hollow configuration causes also a remarkable lightening of the helmet 1.

Always in order to make the removal of exhausted air from the environment 20 more effective, and as best seen in Figure 1D, in the present example a bulkhead 4 is provided positioned within the longitudinal upright 21, and in particular interposed between the two ventilation means 31 and 32 so as to partition the internal portion of the main body 2 into two chambers for air circulation. In the present example, the bulkhead 4 is substantially in the form of a transverse baffle.

As mentioned hereto, the bulkhead 4 allows to carry out a partitioning of the compartment internal to the main body 2 into two chambers, and accordingly a partitioning of the environment 20 into two distinct regions, respectively a delivery region in direct communication with the inletting means 31 and a suction region in direct communication with the suction means 32.

Therefore, the overall configuration obtained with the arrangement of the ventilation means 31 and 32, the bulkhead 4 and the openings 51-53 is such that air is inlet inside the environment 20 by the means 31 and through the longitudinal upright 21 and the front opening 53 of the latter, and then conveyed toward the front zone defined by the mask 24. Suction through the means 32 occurs by the rear part of the longitudinal upright 21, the crosspieces 22 and 23 and the openings 51 and 52 thereof. Thus, the compartment internal to the main body 2, and accordingly the environment 20, is substantially subdivided into a top chamber (delivery air) and a bottom chamber (suction air).

It will be appreciated that the placement of the opening or slit 53 of the longitudinal upright 21 directly at the lens 200 allows a controlled inletting of air directly on the latter, opposing in a maximally effective way its fogging.

Referring now also to Figures 3A and 3B, the helmet 1 comprises means for fitting the main body 2 on the operator's head, and in particular a substantially cap-shaped flexible structure 6 that, in use, is just interposed between the main body 2 and the head.

In particular, the structure 6 comprises a top member 603 which preferably provides a double curvature (spherical and elliptical) for improved fitting to the subject's head.

The structure 6 further provides a longitudinal member 61 for azimuthal adjustment, equipped with a longitudinal toothing 610 or an equivalent engagement means, allowing adjustment of the longitudinal (azimuthal) position of the main body 2 with respect to the health operator's head.

The structure 6 further comprises a pair of circumferential adjustment members, and in particular a left-side member 62 and a right-side member 63, each equipped with a transversal toothing 620, 630 substantially orthogonal, in use, to the longitudinal toothing 610, or with equivalent engagement means. The elements 62 and 63 allow an adjustment of the extension of the base circumference, just to allow the fitting of the helmet 1 to the specific anthropometry of the subject wearing it on.

This twin adjustment option for positioning the surgical helmet 1 on the head increases the stability, in use, of the helmet itself and greatly improves the operator's comfort.

Advantageously, both the circumferential and the azimuthal adjustments are obtainable by a single knob 65, or an equivalent adjustment means, arranged, in use, at the occipital portion of the skull, and equipped with a toothed spindle 650 engaging, in use, the toothings 610 and 620, 630. The spindle 650 may be made with a single module and pitch or with a double module and pitch.

The knob 65 is coupled to the toothings 610 and 620, 630 just by the toothing of the spindle 650. Moreover, it is provided the interposition of a partitioning member 64, operating a partition and allowing a sliding between the azimuthal and circumferential adjustment members 61 and 62, 63. The partitioning member 64 is equipped with a through hole that is crossed by the toothed spindle 650. Moreover, always between toothings 610 and 620, 630 and knob 65, an elastic arresting member 66 is provided that carries out just the arresting and the keeping of the desired position, locking a further sliding of the toothings 610 and 620, 630 on the spindle 650. Such elastic member 66 may provide an axial bending or a circumferential bending and be made, e.g., of plastics.

To the ends of adjustment, the knob 65 is merely rotated to the desired level of azimuthal and circumferential adherence of the structure 6 to the head.

The structure 6 is made integral to the main body 2 at selected points, in particular in the present example in correspondence of two front connection members 601 and 602 and two rear connection members 604 and 605. Furthermore, the structure 6 is completed by a front member 67 inside which the front portion of the azimuthal adjustment member 61 and the two circumferential adjustment members 62 and 63 engage.

It will be appreciated that the hereto-described surgical helmet 1 is particularly susceptible of a modular construction.

For this purpose, in Figure 1A a shield or partializing member 7 is depicted, apt to be constrained, preferably removably, to the main body 2 at the longitudinal upright 21 thereof, so as to exclude the air inletting means 31. In such a configuration, inside the environment 20 a forced air circulation is provided, associated just to the sole suction operated by the means 32.

Figures 4A and 4B refer to a second preferred embodiment of the protection device or surgical helmet of the invention, in this case generally denoted by 11. The helmet 11 differs from the first embodiment described above only in that it provides a single ventilation means, denoted by 33 and arranged in this case as well at a longitudinal upright 21 of a main body 2.

As illustrated hereto for a variant embodiment of the first embodiment, such ventilation means 33 may serve as sole forced air circulation member, or alternatively as inletting and suction member.

Moreover, at the rear base of the main body 2 a bulkhead or an equivalent substitute member may be provided for separating the inlet air volume from the outlet one.

In this case as well, a variant embodiment may be provided in which the ventilation means 33 is entirely or partially placed in a remote position with respect to the main body 2 and in fluid communication with the environment 20 by a vent or sleeve, denoted herein by 303, obtained on or associated to the main body 2.

Figure 5 shows the above-described protection device 11 worn on by a health operator as part of a protection system 100 comprising also the hereto-mentioned lens 200 and the above-cited cap- or robe-type garment, denoted herein by 201, to which the lens itself is associated.

While, as mentioned, Figure 5 shows a surgical helmet 11 complying with the second embodiment, the description thereof, and in particular of the system 100, is also applicable in connection to the first embodiment of the protection device and any variant thereof, as well as in connection to the embodiments that will be described hereinafter.

Preferably, the lens 200 is removably constrainable to the main body 2 of the helmet 1, 11 by Velcro® strips, magnetic members or equivalent means arranged in selected positions on the same helmet and lens. In use, the lens 200 is arranged abutted onto the front mask 24 of the main body 2. Preferably, the overall arrangement is such that, in said operating configuration, the lens is tilted toward the subject with respect to the virtual vertical passing by the base of the main body 2 (or chin guard) according to an angle α, the latter preferably comprised in a range of about 3-8 degrees.

Moreover, preferably additional removable connection means are provided to constrain the main body 2 to the garment 201. Advantageously - and as already anticipated above - in the present example such additional means are based on a shape coupling between the connection flanges 301, 302 or 303 of the main body 2 and corresponding complementary members associated to the garment 201. In the present example, these latter members are in the form of a circular crown.

Figure 6 refers to a third embodiment of the surgical helmet of the present invention, which will be described only in connection with the aspects differentiating it from the above-considered embodiments and variants.

The difference is associated to the different configurations of the housings receiving the first and second ventilation means, here as well designated by 31 and 32. In particular, at the level of the inletting means 31 it is provided the presence of a recess 311 in the profile of the top upright, denoted herein by 210, with the aim of making a sort of air tank or reservoir between the cap or the like covering the helmet and the blower or equivalent means implementing the means 31, and this to the end of an improvement of the efficiency of the latter.

Instead, at the level of the suction means 32 a substantially flat profile 320 is provided, to guarantee adherence between filter and blower discharge.

Figure 7 refers to a fourth embodiment of the surgical helmet of the present invention, which in this case as well will be described merely in connection to the aspects differentiating it from the above-considered embodiments and variants. As already anticipated, in this case the forced circulation means is at least partially housed in a remote position and in particular at the subject's waist by a belt 400. A tube-type or equivalent connector 15 is provided, which sets in fluid communication the suction means, denoted herein by 322, generating the flow of air under suction with the internal chamber of the main body 2.

Moreover, a coupling member 402 in the form of a connector or the like is provided between pipe 401 and main body 2. Preferably, the member 402 is of removable and interchangeable type, also in order to allow operation with the sole delivery (inlet) air for the surgical helmet, in that sense guaranteeing the full modularity and versatility of use of the system.

In the present example, the insertion of a filtering member 403 for virus and bacteria is also provided, interposed between connecting element 402 and tube 401. Variant embodiments may provide that one or more of said filtering members be (also) applied at the level of the above-introduced bushings or flanges of the main body. Integration of such filtering members on the helmet may occur also with the insertion of filtering pockets and/or pleated filters.

Moreover, the remote placement of part of the forced circulation means may also be carried out, e.g., at the level of the shoulders - with a schoolbag-type configuration - or in a different position.

By now, it will be better appreciated that each of the above-described embodiments, variants and configurations allows an optimal conveying and evacuation toward the outside of the exhausted air in the environment accommodating the operator's head, with significant benefits associated to the operator's comfort and to his/her improved vision of the operating field.

Moreover, it will be appreciated that the proposed system is susceptible of a modular construction, thereby enabling the health operator to choose a solution customized and subjectively best for him/herself, guaranteeing head comfort and freedom of body motions.

Said modularity also allows, at the production stage, to employ the same structural members described above with reference to the main body of the helmet for making devices equipped with single or double inlet delivery and/or single or double suction means, employing in that sense the same stationary frame to incorporate different ventilation means.

It will also be better appreciated that the lightness of the helmet is guaranteed by the presence of a load-bearing structure with the abovementioned piping function, wherein each structural volume is hollow and utilized for conveying air under delivery and under suction.

Finally, it has to be noted that for the additional feature related to the presence of removable connection means between main body and lens-bearing garment as defined in the dependent claims and as described above, a protection independent of the presence of the means for forced circulation of air of which at the independent claim might be sought.

Likewise, a separate protection, independent of the presence of the air suction means, might be sought for the piping-type embodiment of the load-bearing structural members forming the main body, as defined in the dependent claims and as described above.

The present invention has been hereto described with reference to preferred embodiments thereof. It is understood that other embodiments might exist, all falling within the concept of the same invention, and all comprised within the protective scope of the claims hereinafter.

## Claims

1. A protection device (1) apt to be worn by a health operator during surgery, comprising:
- a main body (2) substantially shaped as a bearing helmet, apt to surround the health operator's head and to be worn jointly to a transparent vision element (200); and
- means (3) for forced circulation of air in an environment (20) apt to accommodate the operator's head and defined by said main body (2) and by the associated vision element (200),
wherein said forced circulation means (3) comprises air suction means (32), apt to cause an evacuation of exhausted air from said environment (20),
**characterized in that** said main body (2) is internally hollow and **in that** said forced circulation means (3) comprises one or more partitioning bulkheads, apt to partition the internal recess of said main body (2) into a delivery chamber and a suction chamber.

2. The protection device (1) according to claim 1, wherein said air suction means (32) is at least partially housed at said main body (2).

3. The protection device (1) according to the preceding claim, wherein said air suction means (32) is arranged at an apical portion (21) of said main body (2).

4. The protection device (1) according to any one of the preceding claims, wherein said air suction means (32) is housed at a longitudinal upright (21) of said main body (2).

5. The protection device (1) according to any one of the preceding claims, wherein said air suction means (32) is at least partially positionable in a remote position with respect to said main body (2).

6. The protection device (1) according to any one of the preceding claims, wherein said air suction means (32) comprises a connecting element (302; 303) arranged at said main body (2) and apt to establish a fluid communication with said environment (20), wherein preferably said connecting element (302; 303) in the form of vent or sleeve.

7. The protection device (1) according to the preceding claim, wherein said connecting element (302; 303) is arranged at a top portion of said main body (2).

8. The protection device (1) according to any one of the preceding claims, wherein said forced circulation means (3) also comprises means (31) for inletting air in said environment (20).

9. The protection device (1) according to the preceding claim, wherein said air inletting means (31) is implemented as ventilation means distinct from said air suction means (32).

10. The protection device (1) according to claim 8, wherein said air inletting means (31) and said air suction means (32) are implemented by common ventilation means.

11. The protection device (1) according to any one of the preceding claims, wherein said main body (2) defines one or more chambers for delivery and/or suction of air circulated by said forced circulation means (3).

12. The protection device (1) according to any one of the preceding claims, wherein said delivery chamber and suction chamber are respectively a top chamber and a bottom chamber.

13. The protection device (1) according to any one of the preceding claims, wherein said bulkhead or at least one of said bulkheads (4) is arranged at a top portion of said main body (2).

14. The protection device (1) according to any one of the preceding claims, wherein said main body (2) is formed by one or more load-bearing structural members (21-24) having a tubular configuration and apt to guide an air flow generated by said forced circulation means (3) inlet to and/or outlet from said environment (20).

15. The protection device (1) according to the preceding claim, wherein said load-bearing structural members having a tubular configuration comprise one or more transversal uprights (22, 23) and/or a longitudinal upright (21) of said main body (2).

16. The protection device (1) according to any one of the preceding claims, wherein said forced circulation means comprises one or more openings (51-53) for delivery and/or suction of air, obtained at said main body (2) and preferably arranged at a side (22, 23) and/or front (24) portion of the latter.

17. The protection device (1) according to the preceding claim, wherein one or more of said openings (51, 52) are arranged at a chin guard portion (24) of said main body (2).

18. The protection device (1) according to any one of the preceding claims, comprising control means (9) for controlling the flow rate and/or the velocity of air sucked and/or inlet by said forced circulation means (3).

19. The protection device (1) according to any one of the preceding claims, comprising means (301, 302; 303) for the removable connection to a garment (201) associated to the vision element (200), which removable connection means (301, 302; 303) is arranged at said main body (2), and preferably at a top portion of the latter.

20. The protection device (1) according to the preceding claim, wherein said removable connection means is in the form of one or more shaped connection flanges (301, 302; 303).

21. The protection device (1) according to claim 19 or 20, wherein one or more virus- and/or bacteria-filtrating elements are provided, applied at said removable connection means (301, 302; 303).

22. The protection device (1) according to any one of the preceding claims, comprising means (61) for adjusting the longitudinal (azimuthal) position of said main body (2) on the operator's head.

23. The protection device (1) according to any one of the preceding claims, comprising means (62) for adjusting the circumferential position of said main body (2) on the operator's head.

24. The protection device (1) according to claims 22 and 23, wherein said means (61, 62) for adjusting the longitudinal and circumferential position comprise a single actuation means (65) for the simultaneous actuation of said adjusting, which actuation means is preferably in the form of a knob (65).

25. The protection device (1) according to the preceding claim, wherein said actuation means (65) is arranged at an occipital region of the device itself.

26. The protection device (1) according to any one of the preceding claims, comprising means for securing said main body (2) to the vision element (200), which means is of magnetic type.

27. A protection system (100) comprising a protection device (1) according to any one of the preceding claims, a transparent vision element (200) associated or associable to the main body (2) of said device, and connection means between said main body (2) and said vision element (200).

28. The protection system (100) according to the preceding claim, wherein the arrangement is such that, in use, said vision element (200) is tilted of an angle comprised in a range of about 3-8 degrees toward the operator with respect to the vertical.

## Patentansprüche

1. Schutzvorrichtung (1) zum Tragen durch einen Operateur im Gesundheitswesen während einer Operation, umfassend:
- einen Hauptkörper (2), der im Wesentlichen als ein zu tragender Helm geformt ist und geeignet ist, um den Kopf des im Gesundheitswesen tätigen Operateurs zu umgeben und um gemeinsam mit einem transparenten Sichtelement (200) getragen zu werden; und
- Mittel (3) für eine Zwangszirkulation von Luft in einer Umgebung (20), die geeignet ist, den Kopf des Operateurs zu beherbergen und durch den Hauptkörper (2) und durch das zugeordnete Sichtelement (200) definiert ist,
wobei die Zwangszirkulationsmittel (3) Luftansaugmittel (32) umfassen, die geeignet sind, eine Evakuierung von ausgeblasener Luft aus der Umgebung (20) zu verursachen,
**dadurch gekennzeichnet, dass** der Hauptkörper (2) innen hohl ist und dass die Zwangszirkulationsmittel (3) einen oder mehrere unterteilende Querwände aufweisen, die geeignet sind, die interne Ausnehmung des Hauptkörpers (2) in einer Förderkammer und eine Ansaugkammer zu unterteilen.

2. Schutzvorrichtung (1) nach Anspruch 1, wobei die Luftansaugmittel (32) wenigstens teilweise in dem Hauptkörper (2) untergebracht sind.

3. Schutzvorrichtung (1) nach dem vorhergehenden Anspruch, wobei die Luftansaugmittel (32) an einem apikalen Abschnitt (21) des Hauptkörpers (2) angeordnet sind.

4. Schutzvorrichtung (1) nach einem der vorhergehenden Ansprüche, wobei die Luftansaugmittel (32) an einem longitudinalen Stiel (21) des Hauptkörpers (2) angeordnet sind.

5. Schutzvorrichtung (1) nach einem der vorhergehenden Ansprüche, wobei die Luftansaugmittel (32) wenigstens teilweise in einer entfernten Position mit Bezug auf den Hauptkörper (2) positionierbar sind.

6. Schutzvorrichtung (1) nach einem der vorhergehenden Ansprüche, wobei die Luftansaugmittel (32) ein Verbindungselement (302;303) umfassen, dass in dem Hauptkörper (2) angeordnet ist und geeignet ist, eine Fluidkommunikation mit der Umgebung (20) aufzubauen, wobei das Verbindungselement (302;303) vorzugsweise die Form eines Lochs oder eines Schlauchs aufweist.

7. Schutzvorrichtung (1) nach dem vorhergehenden Anspruch, wobei das Verbindungselement (302;303) an einem oberen Abschnitt des Hauptkörpers (2) angeordnet ist.

8. Schutzvorrichtung (1) nach einem der vorhergehenden Ansprüche, wobei die Zwangszirkulationsmittel (3) auch Mittel (31) zum Einlassen von Luft in die Umgebung (20) umfassen.

9. Schutzvorrichtung (1) nach dem vorhergehenden Anspruch, wobei die Einlassmittel (31) als Ventilationsmittel implementiert sind, die sich von den Luftansaugmitteln (32) unterscheiden.

10. Schutzvorrichtung (1) nach Anspruch 8, wobei die Lufteinlassmittel (31) und die Luftauslassmittel (32) durch gemeinsame Ventilationsmittel implementiert sind.

11. Schutzvorrichtung (1) nach einem der vorhergehenden Ansprüche, wobei der Hauptkörper (2) eine oder mehrere Kammern zum Fördern und/oder Ansaugen von Luft definieren, die durch die Zwangszirkulationsmittel (3) zirkuliert wird.

12. Schutzvorrichtung (1) nach einem der vorhergehenden Ansprüche, wobei die Förderkammer und die Ansaugkammer eine obere Kammer beziehungsweise eine untere Kammer sind.

13. Schutzvorrichtung (1) nach einem der vorhergehenden Ansprüche, wobei die Querwand oder wenigstens eine der Querwände (4) an einem oberen Abschnitt des Hauptkörpers (2) angeordnet ist/sind.

14. Schutzvorrichtung (1) nach einem der vorhergehenden Ansprüche, wobei der Hauptkörper (2) durch ein oder mehrere lastaufnehmende Bauteile (21-24) gebildet ist, die eine rohrförmige Konfiguration aufweisen und geeignet sind, einen Luftstrom, der durch die Zwangszirkulationsmittel (3) erzeugt wurde, in das und/oder heraus aus der Umgebung (20) zu leiten.

15. Schutzvorrichtung (1) nach dem vorhergehenden Anspruch, wobei die lastaufnehmenden Bauteile mit einer rohrförmigen Konfiguration eine oder mehrere transversale Querwände (22,23) und oder eine longitudinale Querwand (21) des Hauptkörpers (2) umfassend.

16. Schutzvorrichtung (1) nach einem der vorhergehenden Ansprüche, wobei die Zwangszirkulationsmittel eine oder mehrere Öffnungen (51-53) zum Fördern und/oder Ansaugen von Luft umfassen, aufgenommen in dem Hauptkörper (2) und vorzugsweise an einem Seitenabschnitt (22,23) und/oder Vorderabschnitt (24) des Letzteren angeordnet.

17. Schutzvorrichtung (1) nach dem vorhergehenden Anspruch, wobei eine oder mehrere der Öffnungen (51,52) an einem Kinnschutzabschnitt (24) des Hauptkörpers (2) angeordnet sind.

18. Schutzvorrichtung (1) nach einem der vorhergehenden Ansprüche, mit Steuermitteln (9) zum Steuern der Flussrate und/oder der Geschwindigkeit von durch die Zwangszirkulationsmittel (3) angesaugter und/oder eingelassener Luft.

19. Schutzvorrichtung (1) nach einem der vorhergehenden Ansprüche, mit Mitteln (301,301;303) für die entfernbare Verbindung mit einem Kleidungsstück (201), dass dem Sichtelement (200) zugeordnet ist, wobei die entfernbaren Verbindungsmittel (301,302;303) an dem Hauptkörper (2) und vorzugsweise an einem oberen Abschnitt des Letzteren angeordnet sind.

20. Schutzvorrichtung (1) nach dem vorhergehenden Anspruch, wobei die entfernbaren Verbindungsmittel die Form von einem oder mehreren geformten Verbindungsflanschen (301,302;303) aufweisen.

21. Schutzvorrichtung (1) nach Anspruch 19 oder 20, wobei ein oder mehrere Virus- und/oder Bakterienfilterelemente vorgesehen sind, die an dem entfernbaren Verbindungsmitteln (301,302;303) angelegt sind.

22. Schutzvorrichtung (1) nach einem der vorhergehenden Ansprüche, mit Mitteln (61) zum Einstellen der longitudinalen (azimutalen) Position des Hauptkörpers (2) auf dem Kopf des Operateurs.

23. Schutzvorrichtung (1) nach einem der vorhergehenden Ansprüche, mit Mitteln (62) zum Einstellen der Umfangsposition des Hauptkörpers (2) auf dem Kopf des Operateurs.

24. Schutzvorrichtung (1) nach Anspruch 22 und 23, wobei die Mittel (61,62) zum Einstellen der longitudinalen Position und der Umfangsposition ein einziges Antriebsmittel (65) für den gleichzeitigen Antrieb des Einstellens umfassen, wobei die Antriebsmittel vorzugsweise die Form eines Knopfs (65) aufweisen.

25. Schutzvorrichtung (1) nach dem vorhergehenden Anspruch, wobei die Antriebsmittel (65) in einem okzipitalen Bereich der Vorrichtung selbst angeordnet sind.

26. Schutzvorrichtung (1) nach einem der vorhergehenden Ansprüche, mit Mitteln zum Sichern des Hauptkörpers (2) an dem Sichtelement (200), wobei die Mittel vom magnetischen Typ sind.

27. Schutzsystem (100) mit einer Schutzvorrichtung (1) nach einem der vorhergehenden Ansprüche, einem transparenten Sichtelement (200), das dem Hauptkörper (2) der Vorrichtung zugeordnet ist oder zugeordnet werden kann und Verbindungsmitteln zwischen dem Hauptkörper (2) und dem Sichtelement (200).

28. Schutzsystem (100) nach dem vorhergehenden Anspruch, wobei die Anordnung bei Verwendung derart ausgelegt ist, dass das Sichtelement (200) um einen Winkel gekippt ist, der im Bereich von etwa drei bis acht Grad in Richtung des Operateurs mit Bezug auf die vertikale liegt.

## Revendications

1. Dispositif de protection (1) apte à être porté par un professionnel de santé pendant une opération chirurgicale, comprenant :
un corps principal (2) sensiblement conformé en un casque support, apte à entourer la tête du professionnel de santé et à être porté conjointement avec un élément de vision transparent (200) ; et
un moyen (3) de circulation forcée de l'air dans un environnement (20) apte à recevoir la tête de l'opérateur et défini par ledit corps principal (2) et par l'élément de vision associé (200),
dans lequel ledit moyen de circulation forcée (3) comprend un moyen d'aspiration d'air (32), apte à produire une évacuation de l'air s'échappant dudit environnement (20),
**caractérisé en ce que** ledit corps principal (2) est creux de manière interne et **en ce que** ledit moyen de circulation forcée (3) comprend une ou plusieurs cloisons de séparation, apte(s) à séparer la cavité interne dudit corps principal (2) en une chambre de fourniture et une chambre d'aspiration.

2. Dispositif de protection (1) selon la revendication 1, dans lequel ledit moyen d'aspiration d'air (32) est au moins partiellement logé au niveau dudit corps principal (2).

3. Dispositif de protection (1) selon la revendication précédente, dans lequel ledit moyen d'aspiration d'air (32) est agencé dans une partie apicale (21) dudit corps principal (2).

4. Dispositif de protection (1) selon l'une quelconque des revendications précédentes, dans lequel ledit moyen d'aspiration d'air (32) est logé dans un montant longitudinal (21) dudit corps principal (2).

5. Dispositif de protection (1) selon l'une quelconque des revendications précédentes, dans lequel ledit moyen d'aspiration d'air (32) peut être positionné au moins partiellement dans une position éloignée par rapport audit corps principal (2).

6. Dispositif de protection (1) selon l'une quelconque des revendications précédentes, dans lequel ledit moyen d'aspiration d'air (32) comprend un élément de liaison (302; 303) agencé au niveau dudit corps principal (2) et apte à établir une communication fluide avec ledit environnement (20), dans lequel de préférence, ledit élément de liaison (302; 303) se présente sous la forme d'un évent ou d'un manchon.

7. Dispositif de protection (1) selon la revendication précédente, dans lequel ledit élément de liaison (302; 303) est agencé au niveau de la partie supérieure dudit corps principal (2).

8. Dispositif de protection (1) selon l'une quelconque des revendications précédentes, dans lequel ledit moyen de circulation forcée (3) comprend également un moyen (31) pour faire entrer de l'air dans ledit environnement (20).

9. Dispositif de protection (1) selon la revendication précédente, dans lequel ledit moyen d'entrée d'air (31) est mis en oeuvre sous la forme d'un moyen de ventilation distinct dudit moyen d'aspiration d'air (32).

10. Dispositif de protection (1) selon la revendication 8, dans lequel ledit moyen d'entrée d'air (31) et ledit moyen d'aspiration d'air (32) sont mis en oeuvre par un moyen de ventilation commun.

11. Dispositif de protection (1) selon l'une quelconque des revendications précédentes, dans lequel ledit corps principal (2) définit une ou plusieurs chambres pour fourniture et/ou aspiration d'air mis en circulation par ledit moyen de circulation forcée (3).

12. Dispositif de protection (1) selon l'une quelconque des revendications précédentes, dans lequel lesdites chambre de fourniture et chambre d'aspiration sont respectivement une chambre supérieure et une chambre inférieure.

13. Dispositif de protection (1) selon l'une quelconque des revendications précédentes, dans lequel ladite cloison ou au moins l'une desdites cloisons (4) est(sont) agencée(s) au niveau de la partie supérieure dudit corps principal (2).

14. Dispositif de protection (1) selon l'une quelconque des revendications précédentes, dans lequel ledit corps principal (2) est formé par un ou plusieurs éléments structurels porteurs de charge (21 à 24) ayant une configuration tubulaire et apte(s) à guider un écoulement d'air généré par ledit moyen de circulation forcée (3) en entrée et/ou en sortie dudit environnement (20).

15. Dispositif de protection (1) selon la revendication précédente, dans lequel lesdits éléments structurels porteurs de charge ayant une configuration tubulaire comprennent un ou plusieurs montants transversaux (22, 23) et/ou un montant longitudinal (21) dudit corps principal (2).

16. Dispositif de protection (1) selon l'une quelconque des revendications précédentes, dans lequel ledit moyen de circulation forcée comprend une ou plusieurs ouvertures (51 à 53) pour fournir et/ou aspirer de l'air, obtenue(s) au niveau dudit corps principal (2) et agencée(s) de préférence sur une partie latérale (22, 23) et/ou frontale (24) de ce dernier.

17. Dispositif de protection (1) selon la revendication précédente, dans lequel une ou plusieurs desdites ouvertures (51, 52) est(sont) agencée(s) sur une partie de mentonnière (24) dudit corps principal (2).

18. Dispositif de protection (1) selon l'une quelconque des revendications précédentes, comprenant un moyen de commande (9) pour commander le débit et/ou la vitesse de l'air aspiré et/ou entré par ledit moyen de circulation forcée (3).

19. Dispositif de protection (1) selon l'une quelconque des revendications précédentes, comprenant un moyen (301, 302; 303) pour le raccordement amovible à un vêtement (201) associé à l'élément de vision (200), moyen de raccordement amovible (301, 302; 303) qui est agencé au niveau dudit corps principal (2), et de préférence au niveau de la partie supérieure de ce dernier.

20. Dispositif de protection (1) selon la revendication précédente, dans lequel ledit moyen de raccordement amovible se présente sous la forme d'une ou plusieurs brides de raccordement conformée(s) (301, 302; 303).

21. Dispositif de protection (1) selon la revendication 19 ou 20, dans lequel un ou plusieurs éléments de filtrage de virus et/ou de bactéries est(sont) prévu(s), appliqué(s) au niveau dudit moyen de raccordement amovible (301, 302; 303).

22. Dispositif de protection (1) selon l'une quelconque des revendications précédentes, comprenant un moyen (61) pour régler la position longitudinale (azimutale) dudit corps principal (2) sur la tête de l'utilisateur.

23. Dispositif de protection (1) selon l'une quelconque des revendications précédentes, comprenant un moyen (62) pour régler la position circonférentielle dudit corps principale (2) sur la tête de l'opérateur.

24. Dispositif de protection (1) selon les revendications 22 et 23, dans lequel ledit moyen (61, 62) pour régler la position longitudinale et circonférentielle comprend un moyen d'actionnement unique (65) pour l'actionnement simultané dudit réglage, moyen d'actionnement qui se présente de préférence sous la forme d'un bouton (65).

25. Dispositif de protection (1) selon la revendication précédente, dans lequel ledit moyen d'actionnement (65) est agencé au niveau de la région occipitale du dispositif lui-même.

26. Dispositif de protection (1) selon l'une quelconque des revendications précédentes, comprenant un moyen pour fixer ledit corps principal (2) à l'élément de vision (200), moyen qui est de type magnétique.

27. Système de protection (100) comprenant un dispositif de protection (1) selon l'une quelconque des revendications précédentes, un élément de vision transparent (200) associé ou pouvant être associé au corps principal (2) dudit dispositif, et un moyen de raccordement entre ledit corps principal (2) et ledit élément de vision (200).

28. Système de protection (100) selon la revendication précédente, dans lequel l'agencement est tel que, pendant l'utilisation, ledit élément de vision (200) est incliné d'un angle compris dans la plage allant d'environ 3 à 8 degrés vers l'opérateur par rapport à la verticale.
